Europäisches Patentamt

(19) European Patent Office

Office européen des brevets

(11) EP 0 618 791 B1

## (12) FASCICULE DE BREVET EUROPEEN

(45) Date de publication et mention
de la délivrance du brevet:
**05.03.1997 Bulletin 1997/10**

(21) Numéro de dépôt: **93911662.0**

(22) Date de dépôt: **24.12.1992**

(51) Int. Cl.$^6$: **A61K 7/00**, A61K 9/16,
A01N 25/26, B01J 2/06

(86) Numéro de dépôt international:
**PCT/FR92/01238**

(87) Numéro de publication internationale:
**WO 93/12761 (08.07.1993 Gazette 1993/16)**

(54) **PARTICULES SOLIDES COMPLEXES COMPRENANT UNE SUBSTANCE SOLIDE BIOLOGIQUEMENT ACTIVE, LEUR MODE DE PREPARATION ET COMPOSITIONS A USAGE TOPIQUE LES RENFERMANT DESTINEES AU TRAITEMENT DE SURFACES BIOLOGIQUES**

FERTE COMPLEXE-PARTIKELN ENTHALTEND EINE FERTE BIOLOGISCH AKTIVE SUBSTANZ, IHR HERSTELLUNGSVERFAHREN SOWIE ZUSAMMENSETZUNGEN ZUR TOPISCHEN VERWENDUNG

SOLID COMPLEX PARTICLES COMPRISING A BIOLOGICALLY ACTIVE SOLID SUBSTANCE, MODE OF PREPARATION AND COMPOSITIONS FOR TOPICAL USE CONTAINING THEM AND INTENDED TO TREAT BIOLOGICAL SURFACES

(84) Etats contractants désignés:
**BE CH DE ES FR GB IT LI**

(30) Priorité: **27.12.1991 FR 9116265**

(43) Date de publication de la demande:
**12.10.1994 Bulletin 1994/41**

(73) Titulaire: **LVMH RECHERCHE
92752 Nanterre (FR)**

(72) Inventeurs:
• **MEYBECK, Alain
Les Poissons
F-92400 Courbevoie (FR)**

• **BONTE, Frédéric
F-92400 Courbevoie (FR)**

(74) Mandataire: **Portal, Gérard et al
Cabinet Beau de Loménie
158, rue de l'Université
75340 Paris Cédex 07 (FR)**

(56) Documents cités:
**EP-A- 0 306 236          EP-A- 0 324 725
EP-A- 0 377 370          WO-A-89/12392
WO-A-91/01798           FR-A- 2 611 497
US-A- 4 690 825**

**Description**

La présente invention concerne des nouvelles particules complexes comprenant une substance solide biologiquement active, leur mode de préparation et les compositions à usage topique les renfermant destinées au traitement de surfaces biologiques.

On désignera par surfaces biologiques des surfaces vivantes, en particulier la peau, les phanères ou les parties aériennes des végétaux.

Par matière biologiquement active, on entendra toute matière susceptible d'avoir un effet cosmétique, dermatologique ou pharmaceutique dans une composition destinée au traitement de la peau ou des phanères, ou un effet phytosanitaire dans une composition destinée au traitement des parties aériennes des végétaux.

Certains produits actifs utilisables dans le domaine de la cosmétologie ou en thérapeutique ou dans le traitement phytosanitaire des plantes se trouvent à l'état solide. Lorsqu'on cherche à introduire ces produits à l'état solide dans des formulations destinées à être appliquées sur une surface vivante, et dont on attend une action rapide, on rencontre un double problème technologique :

1. il faut assurer la répartition la plus homogène possible du produit au sein du mélange constituant la formule,
2. il faut que le produit actif puisse être libéré suffisamment rapidement pour obtenir l'effet désiré des produits cosmétiques ou dermatologiques sur la peau ou les phanères ou des produits phytosanitaires sur les parties aériennes des végétaux.

Quand on utilise un produit actif solide, il n'est en général pas possible de le diviser assez finement pour qu'il se libère rapidement, et de plus il est en général difficile de répartir des solides en poudre de façon uni forme sur une surface comme celle de la peau.

La demande européenne EP-A-0 324 725 décrit une composition pharmaceutique constituée d'un mélange ordonné de particules, qui présente la caractéristique essentielle d'être constitué d'un support essentiellement soluble dans l'eau et de particules plus petites, qui adhèrent à ce support. Les particules formant le support présentent la caractéristique de se désintégrer en solution ce qui favorise la rapidité de dissolution du produit dans ses conditions d'utilisation.

On connaît divers procédés de fabrication de particules solides contenant un produit actif solide.

En général, il s'agit d'obtenir que le produit actif se libère plus lentement que s'il s'agissait du produit pur.

Ainsi, le brevet français FR-A-2 611 497 décrit des agglomérats de 0,2 à 2 mm comportant en particulier des particules de principes actifs, conçues de manière que la libération des principes actifs soit prolongée lors de leur application sur la peau.

On a également décrit des enrobages de produits pharmaceutiques destinés à ralentir leur absorption par l'organisme, par exemple dans l'article de H. TAKEUCHI et al., publié dans DRUG DEVELOPMENT AND INDUSTRIAL PHARMACY 15 (12) 1999-2016 (1989).

De telles particules ne conviennent pas lorsqu'il s'agit d'obtenir une accélération de la libération des principes actifs sur la peau ou sur les phanères (cheveux, cils et ongles notamment). C'est le cas en particulier pour un produit cosmétique qui ne reste sur la peau que peu de temps, par exemple entre le nettoyage de celle-ci le matin et le démaquillage du soir. C'est le cas également lorsque les principes actifs sont peu solubles dans le sébum ou dans l'humidité cutanée.

La demanderesse a maintenant trouvé de nouveaux produits permettant de résoudre simultanément tous ces problèmes non résolus par l'art antérieur en proposant des produits nouveaux contenant une substance active solide dont la libération sur une surface vivante telle que celle de la peau ou des phanères ou encore sur les parties aériennes des végétaux s'effectue à une vitesse accrue.

De plus, cette libération n'est généralement reproductible d'une application à l'autre, ce qui est avantageux pour obtenir une activité satisfaisante.

La présente invention concerne donc à titre de produits industriels nouveaux des particules complexes incluant une matière active solide. Elle concerne également leur procédé d'élaboration et leur utilisation notamment dans le domaine de la cosmétologie, de la dermatologie et du traitement des plantes, en particulier dans des compositions où la biodisponibilité de la matière active par rapport à une surface vivante se trouve accrue.

Selon une des caractéristiques essentielles, l'objet de la présente invention est de fournir des particules solides complexes composées chacune d'au moins deux substances solides dont l'une au moins est biologiquement active, caractérisées en ce que ladite substance solide biologiquement active est composée d'au moins un produit biologiquement actif, et est régulièrement répartie à la surface d'un grain constitué de l'autre substance solide formant coeur, dite substance support, ledit grain ayant une dimension comprise entre 0,05 et 100 $\mu$m (microns), et en ce que le rapport pondéral entre ladite substance active et ladite substance formant coeur est compris entre environ $10^{-4}$ et 1,5, ce qui équivaut à une répartition pondérale entre lesdites substances d'environ 0,01 pour 99,99 à 60 pour 40.

Selon une caractéristique avantageuse, la dimension moyenne du coeur des particules complexes selon l'invention est comprise entre 0,3 et 30 $\mu$m (microns).

Suivant une autre caractéristique avantageuse, le rapport pondéral précité est compris entre $10^{-4}$ et 0,35.

Ainsi, les particules selon l'invention comprennent un coeur constitué d'une substance solide servant de support à une substance solide biologiquement active.

Dans la suite de la présente description ainsi que dans les revendications, l'expression "substance support" désignera la substance solide formant coeur précitée.

A titre d'exemples de substances support constituant le coeur des particules de l'invention, on citera :

- des polymères synthétiques solides à température ambiante tels que polyamides, polyéthylène, polystyrène, polyacrylates, polyméthacrylates,
- des minéraux tels que talc, mica, séricite, vermiculite,
- des pigments tels qu'oxyde de titane, oxydes de fer,
- des substances organiques peu solubles telles que lauryl-lysine,
- des cires minérales ou végétales à point de fusion de préférence supérieur à 100°C,
- des polymères naturels tels que la cellulose,
- des particules naturelles telles que des parois de levures ou d'euglène.

De tels produits sont disponibles dans le commerce.

A titre de produits constituant la substance solide biologiquement active disposée régulièrement sur les grains de la substance support, on citera toute matière solide biologiquement active pouvant être utilisée dans des compositions dermatologiques ou cosmétologiques ou encore dans des compositions à usage phytosanitaire.

On citera plus particulièrement parmi ces produits actifs des produits tels que :

- des phosphates vitaminiques, en particulier des phosphates de vitamine E ou de vitamine C,
- des succinates de tocophérol, polyoxyéthylénés ou non,
- l'acide glycyrhizinique, ses sels et ses esters solides,
- l'acide glycyrrhétinique, ses sels et ses esters solides,
- des extraits végétaux pulvérulents, notamment les extraits de Scutellaria, Phellodendron, Glycyrrhiza, Morus alba.
- l'acide kojique et ses dérivés solides,
- les ecdystéroïdes, en particulier la β-ecdysone et ses esters solides,
- l'éconazole,
- le Minoxidil®,
- les bactéricides solides pour déodorants.

La couche de substance biologiquement active entourent le coeur de chaque particule selon l'invention peut également être constituée d'un mélange de produits biologiquement actifs.

Les grains de substance support sont de forme quelconque, en particulier ces grains peuvent être de forme sphérique ou cubique ou sous la forme d'un disque ou d'une plaquette ou encore sous une forme tout à fait irrégulière.

La dimension de ces grains est généralement comprise entre 0,05 et 100 $\mu$m, avantageusement entre 0,3 et 30 $\mu$m.

La substance active précitée est répartie régulièrement à la surface des grains de la substance support en une couche relativement fine formant ainsi des particules complexes. Celles-ci peuvent ensemble former une poudre de façon que, dans ladite poudre, le rapport pondéral de la substance active par rapport à la substance support soit compris entre environ $10^{-4}$ et 1,5.

Avantageusement, ce rapport est compris entre $10^{-4}$ et 0,35.

Selon une variante de l'invention, la particule complexe selon l'invention pourra comprendre en outre, avec la substance biologiquement active, un ou plusieurs additifs, notamment destinés à la conservation, à la coloration ou à la modification des propriétés de surface de ladite particule complexe.

L'invention concerne également un procédé pour obtenir les particules selon l'invention.

Le procédé selon l'invention permet en particulier de répartir de façon uniforme la substance active en couche très mince à la surface des particules de substance support qui elles-mêmes sont très fines, puisqu'elles ont un diamètre moyen de 0,05 à 100 $\mu$m, et de préférence de 0,3 à 30 $\mu$m.

Les particules selon l'invention sont avantageusement obtenues dans un procédé mettant en oeuvre une étape d'atomisation d'une suspension de la substance support dans une solution de la substance biologiquement active.

Pour obtenir les particules selon l'invention, on disperse les grains de la substance support dans un liquide contenant au moins une substance biologiquement active soit sous forme dissoute, soit sous forme d'une dispersion très fine, pour obtenir une suspension de la substance support dans ledit liquide, puis on réalise l'atomisation de ladite suspension pour obtenir une poudre de particules constituée de grains de substance support régulièrement recouverts par la substance biologiquement active.

On choisira comme liquide pour réaliser ladite suspension un produit non-solvant de la substance support.

Comme solvant de la substance active, on choisira par exemple de l'eau ou un solvant organique ou un mélange de ces solvants.

Avantageusement, on choisira ce solvant de façon qu'il soit suffisamment volatil pour pouvoir être éliminé lors de l'étape ultérieure d'atomisation.

A titre d'exemples de solvants utilisables pour mettre en solution la substance active, on citera l'eau, le dichlorométhane, ou leurs mélanges avec l'alcool méthylique ou l'alcool éthylique.

Les conditions d'atomisation et éventuellement le choix de la nature de la substance destinée à former le support sont généralement dictés par les considérations suivantes : l'atomisation est réalisée à une température au moins égale au point d'ébullition du solvant et sensiblement inférieure à la température de ramollissement du support.

Lors de l'étape d'atomisation, la suspension est pulvérisée en très fines gouttelettes dans un courant de gaz chaud, porté de préférence à une température supérieure à celle de l'ébullition du solvant de ladite suspension, de façon que le liquide s'évapore rapidement pour que le produit actif solide se dépose instantanément et régulièrement à la surface de la particule.

L'épaisseur désirée du dépôt de la substance active à la surface de la substance support pourra être obtenue, à taille de gouttelette constante, lors de l'atomisation, en jouant sur tes deux paramètres suivants : concentration de la substance active et concentration en particules support.

La suspension des particules support dans la solution de substance active comprendra avantageusement un produit améliorant le mouillage et/ou la dispersion desdites particules en suspension, ce qui favorisera un dépôt régulier et préférentiel de la substance active à la surface des particules de substance support.

A titre d'exemples de tels produits on citera, en particulier des tensioactifs acceptables pour l'application envisagée, par exemple un monolaurate de sorbitan polyoxyéthyléné, tel que le MONTANOX 20, ou des lipides amphiphiles, tels que des lécithines.

Suivant une autre variante du procédé selon l'invention, les particules selon la présente invention peuvent être préparées par un procédé dérivé de celui décrit dans la demande de brevet français déposée le 4 août 1989 sous le n° 89.10565, et publiée sous le numéro FR 2 650 514.

Plus précisément, selon cette variante, les particules selon l'invention peuvent être obtenues à partir d'une suspension, dans un liquide de dispersion, contenant deux populations de particules solides de tailles sensiblement homogènes comprenant respectivement au moins une population de substance support et au moins une population de substance solide biologiquement active, la taille moyenne des particules de substance active étant de préférence inférieure ou égale à environ 0,2 fois la taille moyenne des particules de substance support. Pour obtenir les particules selon la présente invention, la suspension précitée est pulvérisée dans une enceinte, telle que celle d'un appareil d'atomisation, dans des conditions de pression et de température qui permettent d'obtenir l'évaporation rapide du liquide de dispersion et la formation d'une poudre constituée de particules selon l'invention.

Selon une variante avantageuse de l'invention, on pourra introduire dans le produit destiné à être atomisé différents additifs tels que des colorants, des agents destinés à modifier les propriétés de surface de la particule complexe ou des agents de conservation.

Il est apparu, par ailleurs, que les particules complexes selon l'invention permettaient une libération plus rapide de la substance active au contact d'une surface vivante, telle que celle de la peau ou des phanères ou encore celle des parties aériennes des végétaux et qu'elles permettaient, en outre, une libération plus fiable de ladite substance active.

La présente invention concerne donc également un procédé permettant d'accélérer la biodisponibilité d'une substance biologiquement active contenue dans une composition destinée à être mise en contact avec une surface biologique, comprenant l'utilisation, notamment par application sur ladite surface biologique, d'une composition contenant des particules complexes telles que précédemment définies.

Ainsi, selon la présente invention, il est possible d'obtenir un effet cosmétique ou thérapeutique plus rapide au niveau des couches superficielles de la surface biologique. Le même effet d'accélération peut en outre être obtenu pour des compositions pharmaceutiques à usage transcutané. Le degré de pénétration de la substance biologiquement active peut être ajusté en jouant sur la nature des constituants utilisés dans ladite composition, ce qui est bien connu de l'homme de l'art.

Des tests réalisés en laboratoire dans une cellule de diffusion de FRANTZ munie d'une membrane poreuse en fluorure de polyvinylidène, reliée à un système de détection par spectrométrie UV/visible, ont permis de mettre en évidence la libération à vitesse accrue de la substance active par rapport à celle que l'on obtiendrait par simple mélange intime des deux types de poudre.

L'invention concerne également des compositions à usage cosmétique ou dermatologique ou utilisables dans le domaine phytosanitaire comprenant les particules complexes décrites précédemment ou obtenues par le procédé de préparation décrit plus haut.

Il s'agit généralement de compositions se présentant sous forme solide. Mais il peut s'agir également de compositions sous forme liquide dans la mesure où les particules complexes ne sont pas dissoutes par les autres constituants de la composition.

A titre d'exemples de compositions susceptibles de contenir les poudres complexes de l'invention, on citera en particulier tous les produits cosmétiques pour lesquels les conditions d'application sur la peau autorisent la présence de particules ayant les dimensions des particules complexes de l'invention et dans lesquels la présence de produits biologiquement actifs solides est souhaitable.

Il s'agit en particulier de produits se présentant sous forme pâteuse ou solide, par exemple sous forme de poudre, compactée ou non, sous forme de pâte ou sous forme de suspension.

A titre d'exemples de tels produits, on citera les mascaras, les fards à paupières, les fards à joues, les rouges à lèvres, les fards à cils, les fonds de teint, les poudres libres ou pressées.

Parmi les compositions dermatologiques, on citera les poudres antimycotiques, les poudres cicatrisantes, les talcs anti-inflammatoires, les bâtons pour lèvres gercées.

Parmi les compositions à usage phytosanitaire, on citera les produits de traitement des parties aériennes des plantes, en particulier des feuilles et des tiges, sous forme pulvérulente. L'invention est définie par les revendications.

Exemples

Exemple 1

On utilise comme particules support des sphères de polyamide de type Nylon 12 SP 500 (Toray) ayant un diamètre moyen égal à environ 20 $\mu$m, une masse volumique d'environ 1 g/cm$^3$ et une surface spécifique de 0,68 m$^2$/g, sur lesquelles on dépose un phosphate de vitamine C de la manière suivante :

on dissout 10 g de sel de magnésium du phosphate d'ascorbyle ($C_6H_6O_9P$, $\frac{3}{2}$ Mg) (NIKKOL VC PMG$^®$) de masse volumique d'environ 0,5 g/cm$^3$ dans 200 ml d'eau chauffée à 35°C et agitée,
on ajoute 1 g de monolaurate de sorbitan polyoxyéthyléné (MONTANOX 20$^®$),
on ajoute 90 g de Nylon 12 SP 500,
on complète avec de l'eau jusqu'à un poids total de 500 g, on agite le mélange pour disperser la poudre de billes de polyamide et obtenir une suspension homogène,
on pulvérise la suspension en l'injectant dans un atomiseur Drytech, fonctionnant avec de l'air chaud sous une pression de 7 bars avec un réglage de température de 150°C à l'entrée de l'appareil et 75°C à la sortie, ainsi qu'un débit d'injection de la suspension de 0,5 l/h environ.

On obtient ainsi une poudre blanche (P$_1$) constituée de particules qui se révèlent être homogènes en forme et en taille par examen au microscope électronique à balayage. Le phosphate de vitamine C s'est donc déposé de façon régulière en surface des sphères de Nylon dans une proportion en poids d'environ 10 g de produit biologiquement actif pour 90 g de support, réalisant ainsi des particules complexes selon l'invention. L'épaisseur de substance active répartie à la surface des sphères de Nylon, déterminée par le calcul, est d'environ 0,33 $\mu$m.

Exemple 2

On opère comme à l'exemple 1 pour fabriquer des particules complexes selon l'invention comprenant une proportion en poids d'environ 10 g de diglycyrrhizinate de potassium, de masse volumique d'environ 0,7 g/cm$^3$, pour 90 g de sphères de Nylon 12 SP 500.

Exemple 3

On opère comme à l'exemple 1 pour fabriquer des particules complexes selon l'invention comprenant une proportion en poids d'environ 10 g de tocophéryl-phosphate de sodium, de masse volumique d'environ 0,6 g/cm$^3$, pour 90 g de sphères de Nylon 12 SP 500.

Exemple 4

On utilise comme support des particules de mica, de type mica Concorde grade 400, sous forme de plaquettes de taille moyenne de 30 $\mu$m et de surface spécifique de 9,7 m$^2$/g, sur lesquelles on dépose de l'acide 18-$\beta$-glycyrrhétinique en présence de lécithine de soja.
On procède de la manière suivante :

on dissout 8 g de lécithine de soja dans 200 ml de dichlorométhane sous agitation,
on ajoute à cette solution 2 g d'acide 18-$\beta$-glycyrrhétinique sous agitation,
on rajoute à cette solution 90 g de plaquettes de mica,
on complète avec du dichlorométhane jusqu'à un poids total de 500 g,

on agite le mélange pendant 1 h à 30°C pour faciliter la dispersion du mica et obtenir une suspension homogène,

on pulvérise cette suspension en l'injectant dans un atomiseur Drytech, fonctionnant avec de l'air chaud sous une pression de $7.10^5$ Pa (7 bars), avec un réglage de température de 80°C à l'entrée de l'appareil et 45°C à la sortie, ainsi qu'un débit d'injection de la suspension de 5 l/h environ.

On obtient ainsi une poudre blanche dont les particules unitaires se révèlent être homogènes en forme et en taille par examen au microscope électronique à balayage. L'acide 18-β-glycyrrhétinique s'est donc déposé avec la lécithine de façon régulière en surface des plaquettes de mica dans une proportion en poids respectivement d'environ 2 et 8 g pour 90 g de support.

Exemple 5

On opère comme à l'exemple 4 pour fabriquer des particules selon l'invention comprenant une proportion en poids d'environ 2 g d'acide 18-β-glycyrrhétinique et 8 g de lécithine de soja pour 90 g de talc de type Micro talc IT extra, de surface spécifique d'environ 10 $m^2$/g, et dont les particules ont une forme grossièrement parallélépipédique et une taille moyenne de 5 μm (microns).

Exemple 6

On opère comme à l'exemple 1 pour fabriquer des particules complexes selon l'invention comprenant une proportion en poids d'environ 10 g d'extrait sec de Phellodendron pour 90 g de particules de polyamide de forme irrégulière de type Orgasol 2002 UD et de taille moyenne 20 μm. On opère avec une température de sortie du gaz de 70°C.

Exemple 7

On utilise comme support des particules de polyamide (I) de type ORGASOL 2002D NAT COS®, de taille moyenne d'environ 20 μm. Sur ces particules, on dépose un mélange (II) 50:50 en poids d'acide 18-β-glycyrrhétinique (III) et de succinate de tocophérol polyoxyéthyléné (IV).
Pour cela, on opère de la manière suivante :

10 g de IV sont solubilisés dans 800 ml d'eau à 60°C sous agitation, au moyen par exemple d'un agitateur à hélice de type Rayneri.
10 g de III sous forme pulvérulente sont ajoutés à la même température, en maintenant l'agitation, jusqu'à obtention d'une suspension sensiblement homogène. En maintenant toujours les mêmes conditions, on disperse, par petites quantités, 80 g de particules I, et on poursuit l'agitation jusqu'à l'obtention d'une suspension homogène.

On pulvérise alors cette suspension en l'injectant dans un atomiseur de type DRYTECH, fonctionnant avec de l'air chaud sous pression de 6 bars, avec un réglage de température de 221°C à l'entrée de l'appareil et 78°C à la sortie, ainsi qu'un débit d'injection de la suspension de 0,5 l/h environ.
On obtient ainsi une poudre fine ($P_7$) que l'on observe en microscopie électronique à balayage.
Cette observation montre une répartition très régulière des constituants III et IV à la surface des particules I, le constituant IV étant sous la forme d'un film mince, parsemé régulièrement par des cristaux du constituant III.
Par ailleurs, le dosage des constituants III et IV dans la poudre de particules complexes selon l'invention ainsi obtenue reflète exactement les proportions initiales avant atomisation.

Exemple 8

On opère comme à l'exemple 1 pour fabriquer des particules complexes selon l'invention comprenant une proportion en poids d'environ 25 g de sel de magnésium du phosphate d'ascorbyle et 75 g de polyamide de type Nylon 12.

Exemple 9

On opère comme à l'exemple 1 pour fabriquer des particules complexes selon l'invention comprenant une proportion en poids d'environ 5 g de β-ecdysone et 95 g de poudre de cellulose dont le diamètre des grains est compris entre 50 et 150 μm.

Exemple 10

On se propose de comparer dans cet exemple la vitesse de libération du sel de magnésium du phosphate d'ascorbyle à partir de la poudre (P$_1$) de l'exemple 1, avec la vitesse de libération du même produit actif contenu dans un mélange intime (M$_1$) obtenu par broyage avec une hélice rapide d'un mélange de 10 g de sel de magnésium du phosphate d'ascorbyle (C$_6$H$_6$O$_9$P, $\frac{3}{2}$Mg), 1 g de MONTANOX 20$^®$ et 90 g de Nylon 12 SP 500, ces produits étant ainsi dans les mêmes proportions que dans la poudre de particules complexes (P$_1$) de l'exemple 1. On utilise pour cela un mélangeur de type O.M. DIZER$^®$ (Nara Machinery Co.) comprenant sur le fond un rotor à hélice tournant à 1 800 tr/min et sur le côté une hélice tournant à 3 000 tr/min.

L'étude de la libération du principe biologiquement actif est faite dans une cellule de diffusion de FRANTZ munie d'une membrane en fluorure de polyvinylidène hydrophile-lipophile de type DURAPORE$^®$ HVLP 02500 disponible auprès de la Société MILLIPORE, de porosité égale à 0,45 $\mu$m. Le produit (ou le mélange comparatif) est déposé sur la membrane.

Le compartiment inférieur de la cellule contient une "sous-phase" constituée par de l'eau déminéralisée à 37°C agitée par un barreau aimanté. Cette eau reçoit le produit qui diffuse à travers la membrane humide. A intervalles réguliers, on mesure sa densité optique au moyen d'un spectromètre U.V., pour une longueur d'onde de 243 nm. La densité optique est fonction de la concentration du produit actif dissous dans la sous-phase qui est, dans le cas présent, le sel de phosphate d'ascorbyle.

Cinq essais sont effectués dans les mêmes conditions, en appliquant sur la membrane de la cellule de FRANTZ exactement la même quantité, 20 mg, respectivement de la poudre P$_1$ et du mélange comparatif M$_1$. La diffusion, du principe actif à travers la membrane est observée en fonction du temps par la mesure de la densité optique de la sous-phase.

Le tableau I ci-après donne la valeur moyenne des mesures de densité optique en fonction du temps à partir de l'application sur la membrane des produits, respectivement P$_1$ et M$_1$.

TABLEAU 1

| Densités optiques (diffusion du NIKKOL VCPMG$^®$) | | | | | |
|---|---|---|---|---|---|
| Temps (min) | 7'30" | 15' | 30' | 60' | 90' |
| P$_1$ | 1,37 ± 0,23 | 2,12 ± 0,08 | 2,56 ± 0,09 | 2,62 ± 0,16 | 2,62 ± 0,16 |
| M$_1$ | / | 0,866 ± 0,35 | 1,1 ± 0,71 | 0,91 ± 0,37 | 1,50 ± 0,67 |

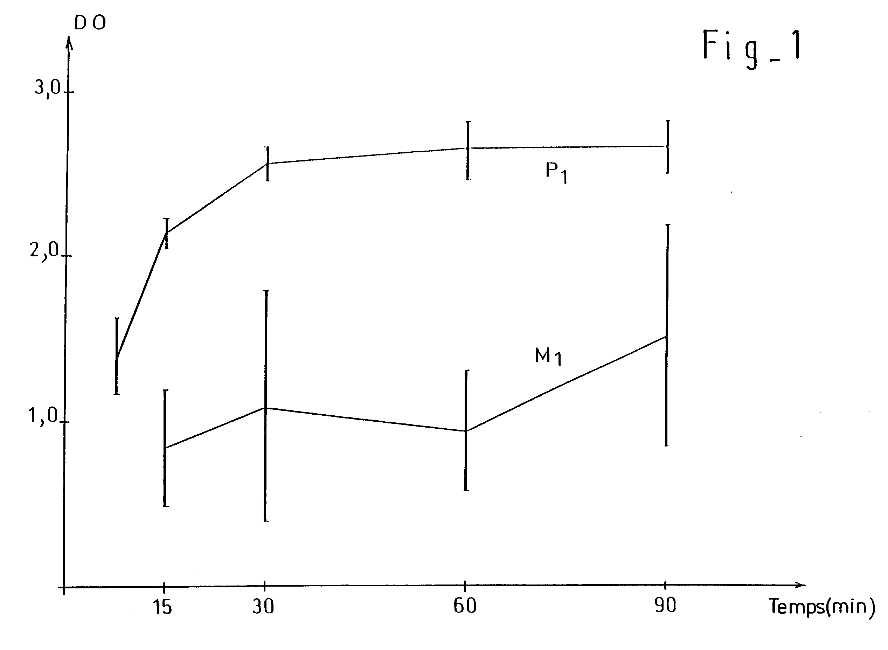

Les résultats ci-dessus sont également représentés sous forme de courbes à la figure 1 qui donne la densité optique (DO) en fonction du temps pour les produits P$_1$ et M$_1$.

A partir du tableau I et de la figure 1, on constate que pour P$_1$ la diffusion du principe actif à travers la membrane est beaucoup plus rapide que pour M$_1$. La quantité de principe actif ayant diffusé est également beaucoup plus importante dès les 30 premières minutes, et même après 90 min. Enfin, on observe que l'écart-type des valeurs moyennes est beaucoup plus important pour M$_1$ que pour P$_1$. Cela signifie que, quels que soient les échantillons de produit utilisés, la diffusion du principe actif est beaucoup plus fiable dans le cas de P$_1$ que dans celui de M$_1$.

Ces résultats obtenus avec la cellule de FRANTZ, qui permet de reproduire, dans des conditions semblables, la libération d'un principe actif appliqué sur la peau ou sur les muqueuses, montrent très clairement que les particules complexes selon l'invention permettent la libération d'un principe actif de manière beaucoup plus rapide, plus efficace (en quantité plus importante), et plus fiable.

## Exemple 11

On opère comme à l'exemple 10, pour comparer le processus de diffusion du succinate de tocophérol polyoxyéthyléné à partir de la poudre de particules complexes P$_7$ de l'exemple 7 d'une part, et à partir du mélange M$_7$ de même composition, à savoir 80 g de particules de polyamide (ORGASOL 2002D$^®$), 10 g d'acide 18-$\beta$-glycyrrhétinique et 10 g de succinate de tocophérol polyoxyéthyléné.

Ce mélange M$_7$ est réalisé de la façon suivante. Le succinate de tocophérol est fondu à 75°C, puis homogénéisé avec un peu d'eau avec les particules de polyamide et l'acide glycyrrhétinique. L'ensemble est mélangé à la spatule jusqu'à mouillage complet de la poudre. Cette poudre est alors séchée, puis broyée au mortier.

Six essais ont été effectués au moyen de la cellule de FRANTZ, pour la poudre P$_7$ d'une part et pour le mélange M$_7$ d'autre part.

On observe la diffusion du succinate de tocophérol polyoxyéthyléné à travers la membrane, dans une sous-phase constituée par de l'eau déminéralisée, en mesurant, comme à l'exemple 10, l'évolution de la densité optique de la sous-phase en fonction du temps, pour une longueur d'onde de 280 nm.

Le tableau II ci-après rassemble les valeurs moyennes sur ces essais des densités optiques mesurées pour P$_7$ et

$M_7$.

TABLEAU II

| Densité optique (diffusion du succinate de tocophérol polyoxyéthyléné) | | | |
|---|---|---|---|
| Temps (min) | 30 | 60 | 90 |
| $P_7$ | $0,038 \pm 0,015$ | $0,061 \pm 0,024$ | $0,082 \pm 0,024$ |
| $M_7$ | $0,0080 \pm 0,005$ | $0,0095 \pm 0,011$ | $0,013 \pm 0,017$ |

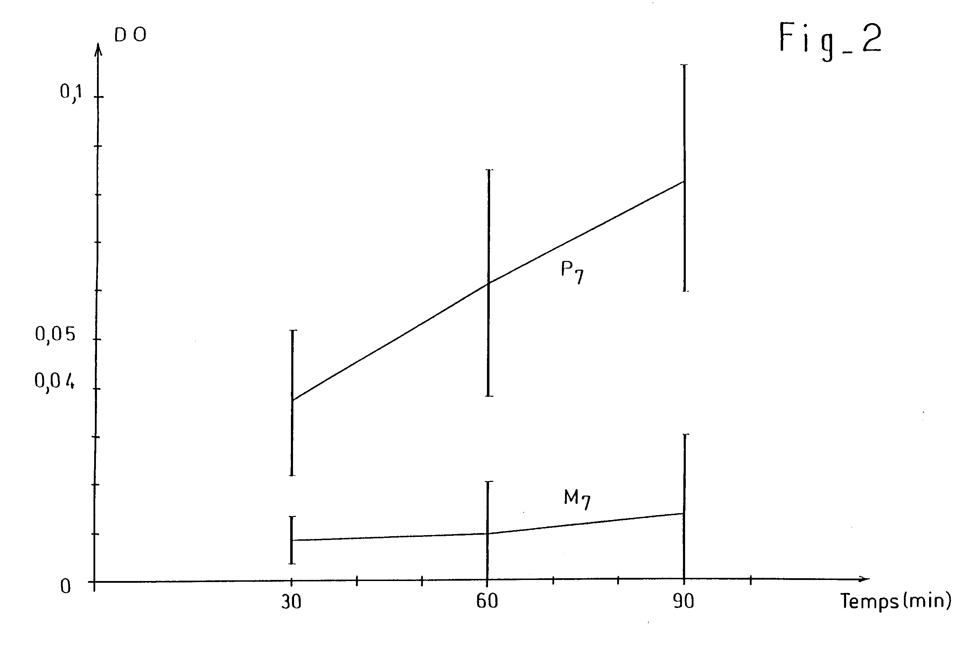

Les résultats ci-dessus sont également représentés sous forme de courbes à la figure 2 qui donne la densité optique (DO) en fonction du temps pour les produits $P_7$ et $M_7$.

A partir du tableau II et de la figure 2, on constate des résultats analogues à ceux obtenus dans le cas de l'exemple 10 précédent. En particulier, on observe que, dans le cas de la poudre $P_7$, la diffusion du principe actif (succinate de tocophérol polyoxyéthyléné) est beaucoup plus rapide, et en quantité beaucoup plus importante que dans le cas du mélange $M_7$.

Par ailleurs, en ce qui concerne l'autre principe actif, constitué par l'acide 18-$\beta$-glycyrrhétinique, oui se présente sous forme de petits cristaux répartis à la surface des particules de polyamide, des essais de diffusion ont également été réalisés dans des conditions similaires à celles exposées plus haut, si ce n'est que la sous-phase est constituée d'un mélange hydroalcoolique à 60 % en volume. La longueur d'onde utilisée pour mesurer la densité optique est ici de 252 nm. Les résultats de ces essais montrent en particulier que les écarts-type des valeurs moyennes de densité optique sont beaucoup plus faibles pour la poudre $P_7$ que pour le mélange $M_7$. Plus précisément, les écarts-type pour $M_7$ sont de 3 à 4 fois plus importants que pour $P_7$, ce qui montre, ici encore, que la diffusion du produit actif est beaucoup plus fiable pour les particules de l'invention que pour le simple mélange.

Exemple 12 :

Bâton traitant pour les lèvres :

Les quantités figurant ci-dessous sont indiquées en parties en poids.

* Phase B

| | |
|---|---|
| Ozokérite | 5,51 |
| Syncrowax BB4 | 7,35 |
| Cire de carnauba | 1,05 |
| Cire de candellila | 2,10 |
| Q 50158 A Wax | 4,68 |
| BHA | 0,02 |
| Nipasol M | 0,05 |
| Crodamol ODL | 6,00 |
| Supermol S | 4,00 |
| Arlamol HD | 14,64 |
| Procas $H_3$ | 10,00 |
| Huile de jojoba | 4,00 |

* Phase A

| Huile de ricin | 30,00 |
|---|---|
| Particules complexes selon l'exemple 3 | 10,00 |

* Phase C

| Glycamil | 0,10 |
|---|---|
| Parfum | 0,50 |

On homogénéise la Phase A par passage dans un broyeur tricylindre.

On ajoute A dans B fondue à 85°C.
On ajoute C dans B + A.
On coule dans des moules préchauffés à 45°C.
On passe 20 min à -5°C.
On attend 15 min à température ordinaire avant le démoulage.

Exemple 13 :

Bâton traitant pour les lèvres :

On opère comme à l'exemple 12 pour réaliser la formule suivante dans laquelle les quantités de produits sont indiquées en parties en poids.

* Phase B

| Ozokérite E 622 | 5,78 |
|---|---|
| Synchrowax BB4 | 7,70 |
| Cire de carnauba | 1,10 |
| Cire de candellila | 2,20 |
| Q 50158 A Wax | 3,85 |
| Phytantriol | 4,95 |
| Supermol S | 4,40 |
| Solulan PB 2 | 1,80 |
| Huile de jojoba | 3,15 |
| Schercemol DID | 5,77 |
| Cosbiol | 4,73 |
| Primol 352 | 3,90 |

\* <u>Phase A</u>

| Huile de ricin | 40,00 |
|---|---|
| Particules complexes de l'exemple 2 | 10,00 |

\* <u>Phase C</u>

| BHA | 0,02 |
|---|---|
| Nipasol M | 0,05 |
| Parfum | 0,60 |
| | $\overline{100,00}$ |

<u>Exemple 14</u> :

<u>Rouge à lèvres adoucissant</u> :

On opère comme à l'exemple 12 avec une Phase B et une Phase C comme à l'exemple 13 et une Phase A de la composition suivante en poids :

| Particules complexes de l'exemple 7 | 5,00 | |
|---|---|---|
| Pigments colorés et couvrants | environ 10 | suivant la teinte |
| Huile de ricin | qsp 50,00 | |

<u>Exemple 15</u> :

<u>Poudre pressée colorée pour paupières sensibles</u> :

On réalise la formule suivante :

| A. | | |
|---|---|---|
| Myristate de magnésium | 4 | |
| Séricite | 22 | |
| Silice | 4 | |
| Nitrure de bore | 2 | |
| Nipagin M | 0,2 | |
| Particules complexes de l'exemple 7 | 10 | |
| B. | | |
| Mélange de pigments et nacres colorées | 15 environ selon la teinte | |
| C. | | |
| Liant | 10 | |
| D. | | |
| Talc | qsp 100 | |

Formule du liant :

| Glycéryl-stéarate | 20 |
|---|---|
| Alcool cétylique | 10 |
| Marcol 82 | 30 |
| Miglyol 812 | 40 |

On mélange A, B et D dans un mélangeur à poudres. On introduit C très progressivement dans le mélange A + B + D .

On presse A + B + D + C en godets à l'aide d'une compacteuse à poudres.

Exemple 16 :

Poudre pressée colorée pour réduire les réactions d'intolérance sur les paupières :

On opère comme à l'exemple 15 pour réaliser des godets de poudre pressée colorée comprenant 10 % de particules complexes selon l'exemple 3.

Exemple 17

Poudre pressée colorée pour le visage permettant d'atténuer la pigmentation naturelle :

On opère comme à l'exemple 15 pour réaliser des godets de poudre pressée comprenant 10 % de particules complexes obtenues selon l'exemple 8.

Exemple 18

Poudre dermatologique cicatrisante

On réalise la formule suivante contenant en parties en poids ;

| - particules complexes obtenues à l'exemple 9 | 20 |
|---|---|
| - excipient pulvérulent à base de cellulose microcristalline | qsp 100 |

Le mélange est réalisé dans un mélangeur à poudres de type classique.

Exemple 19

Formule de mascara crème :

Les pourcentages ci-dessus sont indiqués en pourcentage en poids.

| Veegum | 2 |
|---|---|
| Carboxyméthylcellulose | 0,1 |
| Polynaphtalène sulfonate de sodium | 0,2 |
| Propylèneglycol | 1,4 |
| Cire d'abeilles | 6,5 |
| Huile minérale | 3,5 |
| Pigment noir | 3,00 |
| Acide stéarique | 1,00 |
| Cire de carnauba | 5,00 |
| Particules complexes de l'exemple 7 | 1,00 |
| Conservateur | 0,2 |
| Eau | qsp 100 |

Exemple 20

Poudre pressée pour éclaircir le teint du visage

A) On prépare d'abord des particules solides complexes selon l'invention, conformément au procédé décrit à l'exemple 1, si ce n'est que l'on utilise comme substance active, non pas le sel de magnésium du phosphate d'ascorbyle seul, mais une association à poids égal de ce sel avec un extrait aqueux de mûrier (Morus alba) disponible dans le commerce, notamment chez JAN DEKKER-France. 10 g de cette association sont, comme indiqué dans l'exemple 1, dispersés dans 200 ml d'eau. Les autres constituants utilisés sont les mêmes que ceux indiqués dans cet exemple, avec les mêmes proportions.
B) Les particules solides complexes ainsi préparées sont ensuite incorporées à raison de 4 % en poids dans une formule classique de poudre pressée de maquillage du visage. Grâce aux propriétés dépigmentantes de l'association d'actifs précitée, on obtient ainsi une composition cosmétique traitante sous forme de poudre pressée, destinée à éclaircir le teint, tout en contribuant au maquillage du visage.

**Revendications**

1. Procédé de préparation de particules solides complexes composées chacune d'au moins deux substances solides dont l'une au moins est biologiquement active, ladite substance solide biologiquement active étant composée d'au moins un produit biologiquement actif et étant régulièrement répartie à la surface d'un grain constitué de l'autre substance solide formant coeur, dite substance support, caractérisé en ce que l'on disperse les grains de la substance support ayant une dimension comprise entre 0,3 et 30 μm (microns) dans un liquide contenant au moins une substance biologiquement active soit sous forme dissoute, soit sous forme d'une dispersion dans laquelle les particules de ladite substance biologiquement active ont une taille moyenne inférieure ou égale à 0,2 fois la taille moyenne des particules de la substance support, et en ce que le rapport pondéral entre ladite substance active et ladite substance support est compris entre $10^{-4}$ et 1,5, pour obtenir une suspension de la substance support dans ledit liquide, puis on réalise l'atomisation de ladite suspension pour obtenir une poudre de particules constituée de grains de substance support régulièrement recouverts par la substance biologiquement active.

2. Procédé selon la revendication 1, caractérisé en ce que :

   1) l'on réalise une suspension de particules de substances support dans un liquide contenant au moins une substance biologiquement active sous forme dissoute, ledit liquide n'étant pas solvant de la substance support,
   2) l'on pulvérise ladite suspension en l'injectant dans un atomiseur où l'atomisation est réalisée à une température au moins égale à la température d'ébullition du solvant et inférieure à la température de ramollissement de ladite substance support.

3. Procédé selon la revendication 1, caractérisé en ce que :

   1) l'on réalise une suspension de particules de substances support dans un liquide contenant au moins une substance biologiquement active sous forme d'une dispersion de particules de taille moyenne inférieure ou égale à environ 0,2 fois la taille moyenne des particules de la substance support, ledit liquide n'étant pas solvant de la substance support,
   2) l'on pulvérise ladite suspension en l'injectant dans un atomiseur où l'atomisation est réalisée à une température au moins égale à la température d'ébullition du solvant et inférieure à la température de ramollissement de ladite substance support.

4. Procédé selon l'une des revendications 1 à 3, caractérisé en ce que le rapport pondéral entre la substance active et la substance support est compris entre $10^{-4}$ et 0,35.

5. Procédé selon l'une des revendications 1 à 4, caractérisé en ce que les grains précités formant coeur ont la forme de sphères, de disques, de cubes, de plaquettes ou de grains de formes irrégulières quelconques.

6. Procédé selon l'une des revendications 1 à 5, caractérisé en ce que la substance support est un solide appartenant au groupe des polymères synthétiques solides à la température ambiante, tels que polyamides, polyéthylène, polystyrène, polyacacrylates, polyméthacrylates, des minéraux tels que talc, mica, séricite, vermiculite, des pigments tels qu'oxyde de titane, oxydes de fer, des substances organiques peu solubles dans l'eau, telles que lauryl-lysine, des cires minérales ou végétales, des polymères naturels tels que la cellulose, des particules naturelles telles que des parois de levures ou d'euglène.

7. Procédé selon l'une des revendications 1 à 6, caractérisé en ce que le produit actif appartient au groupe formé des dérivés, par exemple phosphates de vitamine E ou de vitamine C, des succinates de tocophérol, polyoxyéthylénés ou non, de l'acide glycyrrhizinique, de ses sels et esters solides, de l'acide glycyrrhétinique, de ses sels et esters solides, des extraits végétaux pulvérulents, de l'acide kojique, des ecdystéroïdes, de l'éconazole, du minoxidil et des bactéricides solides pour déodorants.

8. Procédé selon l'une des revendications 1 à 7, caractérisé en ce que la suspension introduite dans l'atomiseur contient en outre un produit favorisant le mouillage et/ou la dispersion des particules en suspension.

9. Procédé selon l'une des revendications 7 à 9, caractérisé en ce que le produit destiné à être atomisé contient en outre différents additifs tels que des colorants, des agents destinés à modifier les propriétés de surface de la particule complexe ou des agents de conservation.

**10.** Particules complexes susceptibles d'être obtenues par le procédé de l'une des revendications 1 à 9.

**11.** Utilisation des particules solides complexes susceptibles d'être obtenues selon le procédé de l'une des revendications 1 à 9 pour la préparation d'une composition destinée au traitement d'une surface biologique, telle que la peau, les phanères - en particulier, les cheveux, les cils, les ongles - ou les parties aériennes des végétaux - en particulier, les feuilles et les tiges -, notamment par application sur ladite surface biologique, en vue d'accélérer la biodisponibilité de la substance active vis-à-vis de ladite surface biologique.

**12.** Poudre constituée de particules selon la revendication 10.

**13.** Composition à usage topique destinée au traitement d'une surface biologique telle que la peau, les phanères - en particulier, les cheveux, les cils, les ongles - ou les parties aériennes des végétaux - en particulier, les feuilles et les tiges -, caractérisée en ce qu'elle contient des particules selon la revendication 10.

**14.** Composition selon la revendication 13, caractérisée en ce qu'elle est à l'état solide.

**15.** Composition selon la revendication 13, caractérisée en ce qu'elle est sous forme liquide et en ce que les particules complexes ne sont pas dissoutes par les autres constituants de la composition.

## Claims

**1.** A process for the preparation of solid complex particles each composed of at least two solid substances of which at least one is biologically active , wherein said biologically active substance is composed of at least one biologically active product and is regularly distributed on the surface of a grain constituted by the other solid substance thus forming a core , called support substance, characterised in that the grains of the support substance are dispersed to give a size between 0.3 and 30 microns in a liquid containing at least one biologically active substance be it in solution or be it in suspension in which particles of said biologically active substance have an average size less than or equal to 0.2 times the average size of the support substance particles , and in that the weight ratio between said active substance and said support substance ranges between $10^{-4}$ and 1.5 in order to obtain a suspension of the support substance in said liquid, then said suspension is atomised in order to obtain a powder of particles consisting of grains of support substance uniformly coated with the biologically active substance.

**2.** Process according to Claim 1 , wherein :

1. A suspension of particles of support substances is achieved in a liquid containing at least one dissolved biologically active substance , said liquid not being a solvent of the support substance .
2. Said suspension is pulverised by injecting it into an atomiser wherein the atomisation is achieved at a temperature at least equal to the boiling point of the solvent and less than the softening point of said support substance.

**3.** Process according to claim 1 , wherein :

1. A suspension of particles of support substance is achieved in a liquid containing at least one biologically active substance in the form of a dispersion of particles of average size less than or equal to about 0.2 times the average size of the support substance particles , said liquid not being a solvent of the support substance.
2. Said suspension is pulverised by injecting it into an atomiser where atomisation is achieved at a temperature at least equal to the boiling point of the solvent and less than the softening point of the support substance .

**4.** Process according to Claims 1 to 3 , characterised in that the weight ratio between the active substance and the support substance lies between $10^{-4}$ and 0.35 .

**5.** Process according to Claims 1 to 4 ,characterised in that said grains forming the core are in the form of spheres , disks , cubes , platelets , or grains of any irregular form .

**6.** Process according to Claims 1 to 5 ,characterised in that the support substance is a solid selected from the group consisting of synthetic polymers solid at room temperature , such as polyamides , polyethylene , polystyrene , polyacrylates , polymethacrylates , minerals such as talc , mica , sericite , vermiculite , pigments such as titanium oxide , iron oxides , organic substances sparingly water soluble such as lauryl - lysine , inorganic or vegetable waxes , natural polymers such as cellulose, natural particles such as walls of yeasts or euglene.

7. Process according to one of Claims 1 to 6 , characterised in that the active product is selected from the group consisting of derivatives , for example phosphates of vitamin E or vitamin C , tocopherol succinates polyoxyethylenated or not , glycyrrhizinic acid its salts and its solid esters , pulverulent vegetable extracts , kojic acid , ecdysteroids , econazole , minoxidil , solid bactericides for deodourants .

8. Process according to one of Claims 1 to 7 , characterised in that the suspension introduced into the atomiser contains in addition a product that favours wetting and / or the dispersion of the particles in suspension .

9. Process according to one of Claims 7 or 8 , characterised in that the product to be atomised contains in addition different additives such as colourants , agents that modify the complex particles' surface properties , preservatives.

10. Complex particles susceptible of being obtained by the procedure of one of the Claims 1 to 9 .

11. Use of solid complex particles susceptible of being obtained by the procedure of one of Claims 1 to 9 for the preparation of a composition for treating a biological surface such as the skin , integuments in particular hair, lashes , nails , or aerial parts of plants in particular leaves and stems , notably by application on said biological surface with the aim of accelerating the bioavailability of the active substance towards said biological surface. .

12. Powder consisting of particles according to Claim 10.

13. Composition for topical use for the treatment of a biological surface such as the skin , integuments in particular hair, lashes , nails , or aerial parts of plants in particuliar leaves and stems ,characterised in that it contains particles according to Claim 10 .

14. Composition according to Claim13 characterised in that it is in the solid state.

15. Composition according to Claim 13 characterised in that it is in liquid form and that the complex particles are not dissolved by other constituents of the composition.

**Patentansprüche**

1. Verfahren zur Herstellung komplexer fester Teilchen, die jeweils aus zumindest zwei festen Substanzen bestehen, von denen zumindest eine biologisch aktiv ist, welche biologisch aktive, feste Substanz aus zumindest einem biologisch aktiven Produkt besteht und regelmäßig auf der Oberfläche eines Korns verteilt ist, das aus der anderen festen Substanz besteht, die den Kern bildet, und als Trägersubstanz bezeichnet wird, dadurch gekennzeichnet, daß die Körner der Trägersubstanz mit Abmessungen zwischen 0,3 und 30 μm (Mikrometer) in einer Flüssigkeit dispergiert werden, die zumindest eine biologisch aktive Substanz entweder in gelöster Form oder in Form einer Dispersion enthält, worin die Teilchen der biologisch aktiven Substanz eine mittlere Größe von kleiner oder gleich dem 0,2-fachen der mittleren Größe der Teilchen der Trägersubstanz aufweisen, und daß das Masseverhältnis der aktiven Substanz und der Trägersubstanz zwischen $10^{-4}$ und 1,5 liegt, um eine Suspension der Trägersubstanz in der Flüssigkeit zu erhalten, und anschließend eine Zerstäubung der Suspension durchgeführt wird, um ein Pulver der Teilchen zu erhalten, das aus gleichmäßig mit der biologisch aktiven Substanz bedeckten Körnern der Trägersubstanz besteht.

2. Verfahren nach Anspruch 1, dadurch gekennzeichnet, daß:

1) eine Suspension von Teilchen aus Trägersubstanzen in einer Flüssigkeit hergestellt wird, die zumindest eine biologisch aktive Substanz in gelöster Form enthält, wobei die Flüssigkeit in der Trägersubstanz nicht löslich ist,

2) die Suspension pulverisiert wird, indem sie in einen Zerstäuber eingebracht wird, wo die Zerstäubung bei einer Temperatur zumindest gleich der Siedetemperatur des Lösungsmittels und unter der Erweichungstemperatur der Trägersubstanz durchgeführt wird.

3. Verfahren nach Anspruch 1, dadurch gekennzeichnet, daß:

1) eine Suspension von Teilchen aus Trägersubstanzen in einer Flüssigkeit hergestellt wird, die zumindest eine biologisch aktive Substanz in Form einer Dispersion von Teilchen mit einer mittleren Größe von kleiner oder etwa gleich dem 0,2-fachen der mittleren Größe der Teilchen der Trägersubstanz enthält, wobei die Flüssigkeit in der Trägersubstanz nicht löslich ist,

2) die Suspension pulverisiert wird, indem sie in einen Zerstäuber eingebracht wird, wo die Zerstäubung bei einer Temperatur zumindest gleich der Siedetemperatur des Lösungsmittels und unter der Erweichungstemperatur der Trägersubstanz durchgeführt wird.

4. Verfahren nach einem der Ansprüche 1 bis 3, dadurch gekennzeichnet, daß das Masseverhältnis zwischen der aktiven Substanz und der Trägersubstanz zwischen $10^{-4}$ und 0,35 liegt.

5. Verfahren nach einem der Ansprüche 1 bis 4, dadurch gekennzeichnet, daß die den Kern bildenden Körner die Form von Sphären, Scheiben, Würfeln, Plättchen oder von Körnern mit beliebiger unregelmäßiger Gestalt haben.

6. Verfahren nach einem der Ansprüche 1 bis 5, dadurch gekennzeichnet, daß die Trägersubstanz ein Feststoff ist, der zur Gruppe von bei Umgebungstemperatur festen, synthetischen Polymeren, wie Polyamiden, Polyethylen, Polystyrol, Polyacrylaten, Polymethacrylaten, Mineralien, wie Talkum, Mika, Sericit, Vermiculit, Pigmenten, wie Titanoxid, Eisenoxiden, in Wasser wenig löslichen organischen Substanzen, wie Lauryl-Lysin, Mineral- oder Pflanzenwachsen, natürlichen Polymeren, wie Cellulose, natürlichen Teilchen, wie Wänden von Hefen oder Euglenen, gehört.

7. Verfahren nach einem der Ansprüche 1 bis 6, dadurch gekennzeichnet, daß das aktive Produkt zur Gruppe gehört, die gebildet wird aus Derivaten, beispielsweise Phosphaten von Vitamin E oder Vitamin C, gegebenenfalls polyoxyethylenierten Tocopherolsuccinaten, Glycyrrhizinsäure, ihren Salzen und festen Estern, Glycyrrhetinsäure, ihren Salzen und festen Estern, pulverförmigen Pflanzenextrakten, Kojisäure, Ecdysteroiden, Econazol, Minoxidil und festen Bakteriziden für Deodorantien.

8. Verfahren nach einem der Ansprüche 1 bis 7, dadurch gekennzeichnet, daß die in den Zerstäuber eingebrachte Suspension außerdem ein Produkt enthält, das die Benetzung und/oder Dispersion der Teilchen in Suspension fördert.

9. Verfahren nach einem der Ansprüche 7 bis 9, dadurch gekennzeichnet, daß das zur Zerstäubung bestimmte Produkt außerdem verschiedene Additive, wie Färbemittel, Mittel zur Modifikation der Oberflächeneigenschaften komplexer Teilchen oder Konservierungsmittel, enthält.

10. Komplexe Teilchen, welche durch das Verfahren nach einem der Ansprüche 1 bis 9 erhalten werden können.

11. Verwendung komplexer fester Teilchen, die gemäß dem Verfahren nach einem der Ansprüche 1 bis 9 erhalten werden können, bei der Herstellung einer Zusammensetzung zur Behandlung von einer biologischen Oberfläche, wie der Haut, Hornteilen - insbesondere Haaren, Wimpern, Nägeln - oder an der Luft befindlichen Teilen von Pflanzen - insbesondere Blättern und Stielen -, insbesondere zum Aufbringen auf die biologische Oberfläche zur Beschleunigung der Bioverfügbarkeit der aktiven Substanz in bezug auf die biologische Oberfläche.

12. Pulver, welches aus Teilchen nach Anspruch 10 besteht.

13. Zusammensetzung zur topischen Verwendung bei der Behandlung von einer biologischen Oberfläche, wie der Haut, Hornteilen - insbesondere Haaren, Wimpern, Nägeln - oder an der Luft befindlichen Teilen von Pflanzen - insbesondere Blättern und Stielen -, dadurch gekennzeichnet, daß sie Teilchen nach Anspruch 10 enthält.

14. Zusammensetzung nach Anspruch 13, dadurch gekennzeichnet, daß sie im festen Zustand vorliegt.

15. Zusammensetzung nach Anspruch 13, dadurch gekennzeichnet, daß sie in flüssiger Form vorliegt, und daß die komplexen Teilchen von den anderen Bestandteilen der Zusammensetzung nicht gelöst werden.

Fig _ 1

Fig_2